(19) Europäisches Patentamt
European Patent Office
Office européen des brevets

(11) Publication number: **0 260 814 B1**

# (12) EUROPEAN PATENT SPECIFICATION

(45) Date of publication of patent specification: **05.02.92**

(51) Int. Cl.5: **C07D 233/84**, A61K 31/415

(21) Application number: **87307259.9**

(22) Date of filing: **17.08.87**

Consolidated with 87905838.6/0288488 (European application No./publication No.) by decision dated 13.03.91.

The file contains technical information submitted after the application was filed and not included in this specification

(54) **Dopamine-Beta-hydroxylase inhibitors.**

(30) Priority: **18.08.86 US 898165**

(43) Date of publication of application: **23.03.88 Bulletin 88/12**

(45) Publication of the grant of the patent: **05.02.92 Bulletin 92/06**

(84) Designated Contracting States: **AT BE CH DE ES FR GB GR IT LI LU NL SE**

(56) References cited:
**EP-A- 0 125 033**
**EP-A- 0 125 783**

**CHEMICAL ABSTRACTS, vol. 104, no. 21, 26th May 1986, page 633, abstract no. 186409j, Columbus, Ohio, US; & ZA-A-84 02 962 (SMITHKLINE BECKMAN CORP.) 27-02-1985**

(73) Proprietor: **SMITHKLINE BEECHAM CORPORATION**
**P.O. Box 7929 1 Franklin Plaza**
**Philadelphia Pennsylvania 19101(US)**

(72) Inventor: **Finkelstein, Joseph Alan**
**7549 Brentwood Road**
**Philadelphia Pennsylvania 19151(US)**
Inventor: **Frazee, James Simpson**
**5 Buffalo Run**
**Sewell New Jersey 08080(US)**
Inventor: **Kaiser, Carl**
**1105 Sylvan Drive**
**Haddon Heights New Jersey 08035(US)**
Inventor: **Kruse, Lawrence Ivan**
**33 Firs Walk**
**Tewin Hertfordshire AL6 0NY(US)**
Inventor: **Leonard, Thomas Brent**
**623 Walnut Lane**
**Haverford Pennsylvania 19041(US)**

(74) Representative: **Giddings, Peter John, Dr. et al**
**SmithKline Beecham, Corporate Patents, Mundells**
**Welwyn Garden City, Hertfordshire AL7 1EY(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

This invention relates to inhibitors of dopamine-β-hydroxylase.

### BACKGROUND OF THE INVENTION

In the catecholamine biosynthetic pathway, tyrosine is converted in three steps to norepinephrine (NE). Intermediates are dihydroxyphenylalanine (DOPA) and dopamine (DA). The latter is hydroxylated to norepinephrine by dopamine-β-hydroxylase (DBH) in the presence of oxygen and ascorbic acid.

Inhibition of catecholamine activity has been found to decrease hypertension. See, for example, Matta et al., Clin. Pharm. Ther. 14, 541 (1973), and Teresawa et al., Japan Circ. J. 35, 339 (1971). Weinshilboum, Mayo Clin. Proc. 55, 39 (1980), reviews compounds which inhibit catecholamine activity by interfering with adrenergic receptors. Alternatively, the catecholamine biosynthetic pathway can be suppressed at any of the three steps, resulting in decreased levels of NE. In addition to decreasing hypertension, inhibitors of NE synthesis are active as diuretics, natriuretics, cardiotonics and vasodilators. Inhibition of DBH activity can have the added advantage of increasing levels of DA, which as reported by Ehrreich et al., "New Antihypertensive Drugs," Spectrum Publishing, 1976, pp. 409-432, has been found to have selective vasodilator activity at certain concentrations.

DBH inhibitors have also been shown to reduce or prevent formation of gastric ulcers in rats by Hidaka et al., "Catecholamine and Stress," edit. by Usdin et al., Permagon Press, Oxford, 1976, pp. 159-165 and by Osumi et al., Japan. J. Pharmacol. 23, 904 (1973).

A number of DBH inhibitors are known. These are generally divided into two classes, namely, metal chelating agents, which bind to copper in the enzyme, and phenethylamine analogues. Rosenberg et al., "Essays in Neurochemistry and Neuropharmacology, Vol. 4," edit. by Youdim et al., John Wiley & Sons, 1980, pp. 179-192, and Goldstein, Pharmacol. Rev. 18(1), 77 (1966), review DBH inhibitors. The former report that many of the potent DBH inhibitors have a hydrophobic side chain of size comparable to the aromatic ring of DA, leading the authors to suggest that incorporation of a terminal hydroxyl group on a 4- to 6- carbon side chain on a phenethylamine analogue might yield a potent inhibitor.

Known inhibitors include:

5-alkylpicolinic acids [See, Suda et al., Chem. Pharm. Bull. 17, 2377 (1969); Umezawa et al., Biochem. Pharmacol. 19, 35 (1969); Hidaka et al., Mol. Pharmacol. 9, 172 (1973); Miyano et al., Chem. Pharm. Bull. 26, 2328 (1978); Miyano et al., Heterocycles 14, 755 (1980); Claxton et al., Eur. J. Pharmacol. 37, 179 (1976)];

BRL 8242 [See, Claxton et al., Eur. J. Pharmacol. 37, 179 (1976)];

1-alkyl-2-mercaptoimidazole [See, Hanlon et al., Life Sci. 12, 417 (1973); Fuller et al., Adv. Enzyme Regul. 15, 267 (1976)];

substituted thioureas [See, Johnson et al., J. Pharmacol. Exp. Ther. 168, 229 (1969)]; and

benzyloxyamine and benzylhydrazine [See, Creveling et al., Biochim. Biophys. Acta 64, 125 (1962); Creveling et al., Biochim. Biophys. Res. Commun. 8, 215 (1962); van der Schoot et al., J. Pharmacol. Exp. Ther. 141, 74 (1963). Bloom, Ann. N. Y. Acad. Sci. 107, 878 (1963)].

All of the above compounds except benzyloxyamine and benzylhydrazine apparently owe their inhibitory effect to metal chelating properties. Alkyl derivatives of 2-mercaptoimidazole are more potent, presumably due to non-specific interaction of the alkyl substituent with the enzyme. Benzyloxyamine and benzylhydrazine are phenethylamine derivatives which apparently act as competitive inhibitors.

In addition to the above compounds, Runti et al., Il Farmaco Ed. Sc. 36, 260 (1980), report that other fusaric acid derivatives and analogues inhibit DBH. These include phenypicolinic acid, which is reported to have twice the inhibitory activity of fusaric acid, and 5-(4-chlorobutyl)picolinic acid, and others such as substituted amides of fusaric acid and acids and amides of 5-butyroylpicolinic acid, 5-aminopicolinic acid and 5-hydrazinopicolinic acid, and derivatives thereof.

Hidaka et al., Molecular Pharmacology, 9, 172-177 (1973) report that 5-(3,4-dibromo)butyl picolinic acid and 5-(dimethyldithiocarbamoyl)methyl picolinic acid are DBH inhibitors.

Bupicomide, 5-(n-butyl)picolinamide, is reported by Ehrreich et al., "New Antihypertensive Drugs," Spectrum Publications, 1976, pg. 409-432, to be a DBH inhibitor and to have antihypertensive activity.

Friedman et al. Psychosomatic Med. 40, 107 (1978), report that patients treated with alpha-methyl-DOPA, guanethidine and reserpine, but not propanolol and diuretics, have lowered DBH levels, although the significance of the observation is uncertain.

Although there are many known inhibitors of DBH, most of these agents have not found clinical application because of non-specific, often toxic, properties they possess. Fusaric acid, for example, is hepatotoxic. See, for example, Teresawa et al., Japan. Cir. J. 35, 339 (1971) and references cited therein. Presumably, the picolinic acid structure interacts with a number of metalloproteins and enzymes in non-specific fashion to produce observed side effects.

In U.K. specification 1,555,580 are disclosed compounds having the formula:

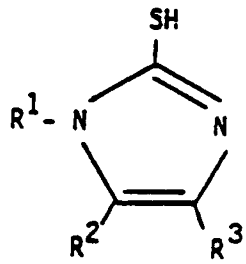

wherein $R^2$ and $R^3$ can be H and $R^1$ can be substituted phenyl. The compounds are said to have analgesic, antiinflammatory and antipyretic properties. Gebert et al., U.S. Patent 3,915,980, disclose such compounds wherein $R^1$ can be phenyl or phen($C_{1-3}$) alkyl, as intermediates to imidazolyl-2-thioalkanoic acid esters.

Iverson, Acta Chem. Scand. 21, 279 (1967) reports a compound having the formula:

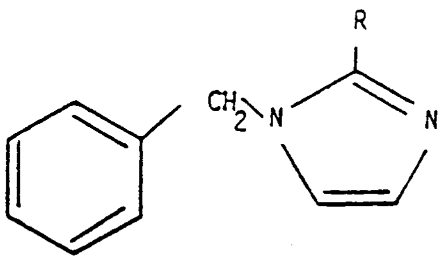

herein R can be $-CO_2H$ or $-CH_2NCH_6H_5$, but does not report a pharmaceutical use for the compound.

In European Patent Application No. 125,033 (published November 14, 1984) a series of 1-phenyl and 1-phenylalkylimidazole compounds having a mercapto or alkylthio group in the 2-position are disclosed. These compounds are described as having DBH inhibiting activity.

In addition, this application discloses a series of such compounds in which the phenyl ring is substituted with a 4-methoxy group. Those compounds are, however, disclosed as chemical intermediates only and no pharmacological activity is attributed to them.

United States Patent No. 4,532,331 describes various 1-benzyl-2-aminomethylimidazole derivatives that inhibit DBH activity and includes pharmaceutical compositions containing these derivatives and methods of using these derivatives to inhibit DBH activity.

## SUMMARY OF THE INVENTION

The invention resides in the discovery that DBH is inhibited by compounds having a mercaptoimidazole moiety and a 4-alkoxyphenalkylamine moiety. More particularly, the invention is pharmaceutical compositions comprising selected compounds having the formula (I):

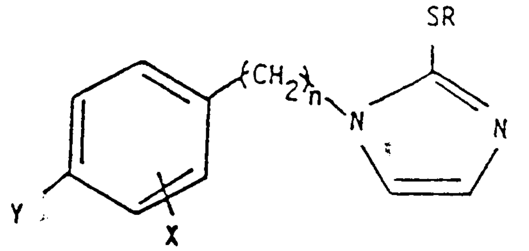

(I)

wherein:

Y is $C_{1-4}$ alkoxy ;

X is -H, -OH, halogen, $C_{1-4}$ alkyl, -CN,-$NO_2$, -$SO_2NH_2$, -$CO_2H$, - $CONH_2$, -CHO, -$CH_2OH$, -$CF_3$, $C_{1-4}$ alkoxy, -$SO_2C_{1-4}$ alkyl, -$SO_2C_{1-4}$ fluoroalkyl, -$CO_2C_{1-4}$ alkyl or any accessible combination thereof up to four substituents;

R is -H or $C_{1-4}$ alkyl; and

n is 0-4,

or a hydrate or, when R is $C_{1-4}$ alkyl, a pharmaceutically acceptable acid addition salt thereof. As used herein, "accessible combination thereof" means any other stable combination of substituents available by chemical synthesis.

In preferred compounds of the invention, Y is -$OCH_3$; R is -H; n is 1 or 3; and X is -H, -OH or halogen (in particular, 3,5-dichloro, 3,5-difluoro, 3-chloro, or 3-fluoro)

The invention is also the use of compounds of formula (I) in therapy, in particular as inhibitors of DBH activity.

Compounds found to be especially potent, and therefore preferred in the method of invention, are those in which Y is $OCH_3$; R is -H; n is 1 or 3; and X is -H, -OH or halogen (in particular, 3,5-dichloro 3,5-difluoro, 3-chloro or 3-fluoro).

It is intended that the above formulae include the tautomer of the compounds wherein R is -H, that is, the compounds having the above formulae wherein the imidazole moiety has the formula:

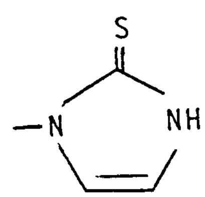

The above formulae also include hydrates of the compounds and pharmaceutically acceptable acid addition salts of the compounds wherein R is $C_{1-4}$ alkyl.

The invention is also a process for preparing certain of the compounds of formula (I) which comprises contacting 1-(3-fluoro-4-methoxybenzyl)aminoacetaldehyde dimethyl acetal or 1-(3,5-difluoro-4-methoxybenzyl)aminoacetaldehyde dimethyl acetal with acidic thiocyanate to form 1-(3-fluoro-4-methoxybenzyl)-2-mercaptoimidazole or, 1-(3,5-difluoro-4-methoxybenzyl)-2-mercaptoimidazo respectively. These two novel compounds comprise the final aspect of the invention.

The compounds of the invention and the compounds used in compositions of the invention can be prepared from corresponding starting benzyl or phenyl compounds such as benzaldehydes, which are known and described in published references or are readily accessible, by known techniques such as illustrated in Scheme I, below, wherein $X^1$ is X, except that when X is -OH, $X^1$ is $C_{1-4}$ alkoxy, preferably -$OCH_3$. As illustrated, n is one, although n can be 0-4. Scheme I illustrates reductive amination of benzaldehydes (I) with an aminoacetaldehyde acetal followed by reduction by, for example, catalytic hydrogenation or treatment with a reducing agent such as $NaBH_4$, $LiAlH_4$ or $AlH_3$, to provide intermediate substituted benzylamines (II). Upon reaction with acidic thiocyanante, the intermediates (II) yield mercaptoimidazole products (III). The mercaptoimidazole products can be prepared from other than benzaldehydes, as illustrated in Examples 1 and 4, below.

**Scheme I**

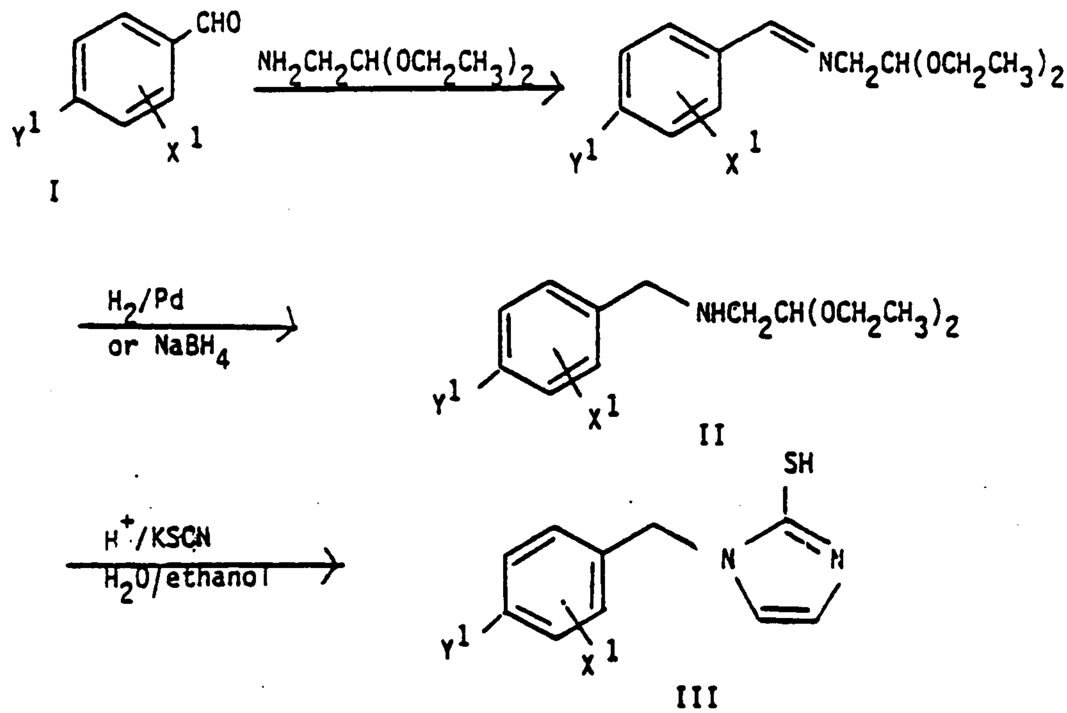


I

II

III

The 1-phenyl substituted 2-mercaptoimidazoles (n is 0) are preferably prepared by reaction of an appropriately substituted phenyl isothiocyanate with an aminoacetaldehyde acetal followed by strong acid catalyzed cyclization, as illustrated in Example 1, below.

The compounds wherein n is 2, 3 or 4 are preferably prepared as illustrated in Example 4 and in Examples 23 and 24, below. Coupling of substituted phenyalkanoic acids as the acid halides, preferably chlorides, with aminoacetaldehyde acetals and subsequent reduction provides such intermediate substituted phenylalkylamines.

$X^1$ in Scheme I is the same as X except that when X is -OH, $X^1$ is $C_{1-4}$ alkoxy, preferably $-OCH_3$, optional deprotection of the alkoxy group with, for example, $BBr_3$ or HBr, or nucleophilic aromatic substitution with dilute hydroxide, provides the phenol (X is -OH). $X^1$ may be one or more substituents at the 2-, 3-, 5- or 6- positions, provided the combination of substituents is accessible, that is, does not result insignificant instability due to steric hindrance.

The compounds in which R is $C_{1-4}$ alkyl are preferably prepared by allowing the deprotection with, for example, $BBr_3$, in an alkanol to proceed to formation of an alkyl bromide which alkylates the mercapto group as illustrated in Example 6, below. Alternatively, a solution or suspension of an appropriately substituted mercaptoimidazole in an inert solvent, for example, methanol, tetrahydrofuran or aqueous dimethylformamide, can be reacted with an alkylating agent, for example, alkyl iodide, bromide or tosylate. Methyl iodide is preferred in this alternative procedure.

The pharmaceutically acceptable acid addition salts of the compounds wherein R is $C_{1-4}$ alkyl are formed with strong or moderately strong organic or inorganic acids by methods known to the art. For example, the base is reacted with an inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of the salts which are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts.

The compounds of the invention, because they can be used to inhibit DBH activity, have therapeutic value as diuretic, natriuretic, cardiotonic, antihypertensive and vasodilator agents, as well as antiulcerogenic and antiparkinson disease agents.

Compounds of the invention and other compounds useful in the inhibition of DBH activity were

screened for in vitro DBH inhibition by a standard procedure for assaying conversion of tyramine to octopamine in the presence of DBH. Octopamine was assayed following sodium periodate oxidation to p-hydroxybenzaldehyde by measuring spectrophotometric absorbance at 330 nm. Results are given in Table I, below. Inhibition is given in molar concentration of compound at which DBH activity was halved ($IC_{50}$). Melting points (mp) are given in °C. By this procedure fusaric acid was found to have an $IC_{50}$ of about $8x10^{-7}$.

TABLE I

| n | X | Y | R | mp | $IC_{50}$ |
|---|---|---|---|---|---|
| 1 | 3-F | OH | H | 172 | $1.4x10^{-6}$ |
| 1 | 3-F | $OCH_3$ | H | 156-157 | $10^{-4}$ (67% inhibition) |
| 1 | 3,5-$F_2$ | OH | H | 213-215 | $7.4x10^{-8}$ |
| 1 | 3,5-$F_2$ | $OCH_3$ | H | 160-161 | $3.6x10^{-5}$ |

The above results illustrate that compounds of the invention and other related compounds disclosed in EP-A-125033 ie. the compounds in which Y is OH, inhibit DBH activity.

Various compounds of the invention, as well as various known DBH inhibitors disclosed in EP-A-125033, were tested for their effects on peripheral dopamine and norepinephrine levels substantially by the procedure of DaPrada and Zürcher, Life Sci. 19, 1161 (1976). Spontaneously hypertensive rats were dosed twice, the second dose being about 18 hr after the first, and were sacrificed about 2 hr after the second dose. Averaged results, expressed in micrograms of DA per gram of tissue, are given in Tables II and in Table II A, which follow. In Table II, R= -H, Y= -OH and n=1; in Table II A, R= -H, Y= $-OCH_3$, and n=1.

TABLE II

| Compound | No. of Animals | DA (µg/g) | | DA/NE Ratio | |
|---|---|---|---|---|---|
| Control ($H_2O$) | 11 | .260 | .019 | .040 | .002 |
| Fusaric Acid | 11 | .520 | .053(1) | .100 | .007(1) |
| Control | 3 | .219 | .044 | .035 | .002 |
| Hydralazine | | | | | |
| (25 mg/kg) | 3 | .417 | .026(2) | .078 | .015(2) |
| (50 mg/kg) | 1 | .835 | .127 | .098 | .018 |
| Control | 1 | .299 | .038 | .039 | .004 |
| X=3-F (50 mg/kg) | 1 | .476 | .023(3) | .064 | .004(2) |
| Control | 1 | .261 | .046 | .041 | .007 |
| X=3-F | | | | | |
| (50 mg/kg) | 1 | .430 | .027(3) | .082 | .005(3) |
| (100 mg/kg) | 1 | .619 | .071(3) | .099 | .003(1) |
| Control | 1 | .273 | .019 | .040 | .002 |
| X=3,5-$Cl_2$ (50 mg/kg) | 1 | .241 | .031(3) | .045 | .006(3) |
| Control (cold-stressed) | 1 | .242 | .014 | .030 | .002 |
| X=3,5-$Cl_2$ (50 mg/kg) (cold-stressed) | 1 | .313 | .019(2) | .038 | .003(2) |

(1) $P < 0.001$ (2) $P < 0.05$ (3) $P < 0.01$

TABLE II (Continued)

| Compound | No. of Animals | DA (μg/g) | | DA/NE Ratio | |
|---|---|---|---|---|---|
| Control | 5 | .270 | .025 | .0307 | .0019 |
| Fusaric Acid (50 mg/kg) | 5 | .675 | .030(1) | .0871 | .0047(1) |
| X=3,5-$F_2$ (50 mg/kg) | 5 | .708 | .068(1) | .0824 | .0128(2) |

(1) $P < 0.001$ (2) $P < 0.05$ (3) $P < 0.01$

TABLE IIA

| Compound | No. of Animals | DA (μg/g) | DA/NE Ratio |
|---|---|---|---|
| Control | 6 | .318 ± .008 | .0393 |
| X=3-F | 6 | 1.397 ± .111 | .239(1) |
| X=3,5-$F_2$ | 6 | 1.178 ± .087 | .204(1) |

(1) P 0.001

The above results illustrate that the compounds of the invention inhibit DBH activity in mammals when administered internally in effective amounts.

In particular, although relatively weak in vitro DBH inhibitors, as indicated by the data in Table IIA, compounds in which Y is $-OCH_3$, n is 1, and X is 3-F or 3,5-$F_2$ proved very potent DBH inhibitors in vivo.

In a study conducted to compare the effects on blood pressure of compounds wherein Y is -OH, X is 3-F or 3,5-$F_2$ and n is 1 to their Y is $-OCH_3$ analogues, the maximum blood pressure reduction observed after administration of the hydroxy compound in which X is 3-F was 18%, whereas one-half the dose of its methoxy analogue produced a 35% blood pressure reduction. Also, equivalent doses of the compound in which Y is -OH, X is 3,5-$F_2$, and n is 1 and its Y is $-OCH_3$ analogue produced, respectively, 20% and 35% blood pressure reductions.

The compounds can be incorporated into convenient dosage unit forms such as capsules, tablets or injectable preparations. Pharmaceutical carriers which can be employed can be solid or liquid. Solid carriers include, among others, lactose, terra alba, sucrose, talc, gelatin, agar, pectin, acacia, magnesium stearate, and stearic acid. Liquid carriers include, among others, syrup, peanut oil, olive oil and water. Similarly, the carrier or diluent may include any delayed release material, such as glyceryl monostearate or glyceryl distearate, alone or with a wax. The amount of solid carrier will vary widely but, preferably, will be from about 25 mg to about 1 g per dosage unit. If a liquid carrier is used, the preparation will be in the form of a syrup, emulsion, soft gelatin capsule, sterile injectable liquid such as an ampule, or an aqueous or nonaqueous liquid suspension.

The pharmaceutical preparations are made following conventional techniques of a pharmaceutical chemist involving mixing, granulating and compressing, when necessary, for tablet forms, or mixing, filling and dissolving the ingredients, as appropriate, to give the desired oral or parenteral end products.

Doses of the present compounds in a pharmaceutical dosage unit will be an effective amount, that is, a nontoxic quantity selected from the range of 0.1-1,000 mg/kg of active compound, preferably 10-100 mg/kg. The selected dose is administered to a patient in need of treatment from 1-5 times daily, orally, rectally, by injection or by infusion. Parenteral administration, which uses a low dose, is preferred. However, oral

administration, at a higher dose, can also be used when safe and convenient for the patient. Use of lowest effective doses is recommended because toxicity has been associated with sulfur-containing compounds.

The following examples are illustrative of preparation of compounds of the invention or intermediates therefor. The starting compounds are commercially available or are prepared by known techniques. The compounds listed in Tables I and II, above, were prepared substantially by the illustrated procedures. All temperatures and melting points (mp) are in degrees Celsius (° C).

EXAMPLE 1

1-(4-Methoxyphenyl)-2-mercaptoimidazole

A solution of 10 g (.06 mole) of p-methoxyphenyl isothiocyanate in 100 ml of $CHCl_3$ was treated with 6.3 g (.06 mole) of aminoacetaldehyde dimethyl acetal. The solvent was evaporated and the residue was recrystallized from ethanol to yield N-(p-methoxyphenyl)-N′-($\beta$,$\beta$-dimethoxyethyl)thiourea, 9.2 g (57%). A suspension of this thiourea in a solution of 5 ml of concentrated $H_2SO_4$ and 20 ml of $H_2O$ was refluxed for 3 hours. The mixture was cooled and a solid was filtered, washed with $H_2O$ and dried. Recrystallization from ethanol gave 1-(4-methoxyphenyl)-2-mercaptomidazole, 4.9 g (70%), mp 215-7°. The compound can be deprotected, for example, as illustrated in Example 5 and 6, below, to prepare the phenol, Y is -OH.

EXAMPLE 2

1-(4-Methoxybenzyl)-2-mercaptoimidazole

A mixture of 13.6 g (0.1 mole) of anisaldehyde, 13.3 g (0.1 mole) of aminoacetaldehyde diethyl acetal and 1 ml of $CH_3OH$ was heated at 95° for 10 minutes. A residue was dissolved in 150 ml of ethanol and hydrogenated over 10% Pd on carbon at 50 psi (0.34 MPa) until $H_2$ uptake was complete. The catalyst was filtered and the filtrate was treated with 10.4 g (0.107 mole) of KSCN, 40 ml of 3N HCl and 40 ml of $H_2O$. The mixture was refluxed, letting the solvent evaporate until the volume of the reaction mixture was 100 ml. After 45 minutes, the mixture was cooled, and a solid was filtered, washed with $H_2O$ and dried. Recrystallization from ethanol gave (1-(4-methoxybenzyl)-2-mercaptoimidazole, 15.0 g (68%), mp 140-142°.

EXAMPLE 3

1-(3-Bromo-4-methoxybenzyl)-2-mercaptoimidazole

A solution of 10.75 g (.05 mole) of 3-bromo-4-methoxybenzaldehyde and 6.65 g (.05 mole) of aminoacetaldehyde diethyl acetal in 25 ml of ethanol was refluxed for 30 minutes. The solvent was evaporated and the residue was dissolved in $CH_2Cl_2$. The $CH_2Cl_2$ solution of the Schiff base was washed with saturated aqueous NaCl, dried ($K_2CO_3$) and filtered, and the solvent was evaporated. Residual Schiff base was dissolved in 100 ml of methanol, cooled to 5°, and treated with 5.0 g of $NaBH_4$. The reaction mixture was allowed to warm to 22° and, after 4 hr, the solvent was evaporated. The residue was taken up in diethyl ether, washed with $H_2O$, dried ($MgSO_4$) and filtered, and the solvent was evaporated. A solution of the residue in $CHCl_3$, upon treatment with ethereal HCl, gave, on standing, crystals of N-(3-bromo-4-methoxybenzyl)aminoacetaldehyde diethylacetal hydrochloride, 10.75 g (58%), mp 112-120°.

A solution of 10.74 g (.029 mole) of N-(3-bromo-4-methoxybenzyl)aminoacetaldehyde diethyl acetal hydrochloride and 3.37 g (0.35 mole) of KSCN in 50 ml of $H_2O$, 50 ml of ethanol and 5 ml of 3N HCl was refluxed for 4.5 hours. One hundred ml of $H_2O$ was added and the mixture was cooled. A solid was filtered, washed with $H_2O$ and dried. Recrystallization from ethanol gave 1-(3-bromo-4-methoxybenzyl)-2-mercaptoimidazole, 6.3 g (72%), mp 188°.

EXAMPLE 4

1-[3-(4-Methoxyphenyl)-propyl]-2-mercaptoimidazole

A solution of 12.5 g (.07 mole) of p-methoxyphenylpropionic acid in 100 ml of $CH_2Cl_2$ and one drop of pyridine was treated with 9.8 g (.077 mole) of oxalyl chloride. After 2.5 hours, the solvents were thoroughly evaporated to give the acid chloride as an oil. A solution of the acid chloride in 100 ml of $CH_2Cl_2$ was slowly added to a cold (0°) solution of 14.7 g (0.14 mole) of aminoacetaldehyde dimethyl acetal in 300 ml

of $CH_2Cl_2$ at a rate such that the temperature stayed below 20°. After 1 hr, the reaction mixture was poured into $H_2O$, and the $CH_2Cl_2$ layer was separated and washed with aqueous $Na_2CO_3$, 0.5N HCl and $H_2O$. Following drying and evaporation of the solvent, N-(β, β-dimethoxyethyl)-p-methoxyphenylpropionamide was obtained as a solid, 10.3 g (55%). A solution of this amide in 300 ml of diethyl ether was slowly added to a slurry of 4.0 g of $LiAlH_4$ in 400 ml of diethyl ether and 350 ml of tetrahydrofuran (THF). After 3.5 hours at 22°, excess $LiAlH_4$ was cautiously destroyed, the reaction mixture was filtered and the filtrate was evaporated. The residue was dissolved in 100 ml of 0.15N HCl, washed with diethyl ether, basified with $NaHCO_3$ and extracted with diethyl ether. The extracts were dried ($MgSO_4$) and the solvent was evaporated to give N-[3-(4-methoxyphenyl)propyl] aminoacetaldehyde dimethyl acetal, 4.6 g (52%), as an unstable oil.

A solution of 3.62 g (.014 mole) of N-[3-(4-methoxyphenyl)propyl]aminoacetaldehyde dimethyl acetal and 1.4 g (.0144 mole) of KSCN in 20 ml of ethanol, 5 ml of $H_2O$ and 2 ml of concentrated HCl was refluxed for five hours. Fifty ml of $H_2O$ was added, the mixture was cooled and a solid was filtered, washed with $H_2O$ and dried. Recrystallization from ethanol gave 1-[3-(4-methoxyphenyl)propyl]-2-mercaptoimidazole, 2.4 g (69%), mp 108-109°.

Example 5

1-(3-Fluoro-4-methoxybenzyl)-2-mercaptoimidazole

3-Fluoro-4-methoxybenzaldehyde (5.0 g, 0.0324 mole) was added to aminoacetaldehyde dimethyl acetal (3.53 ml, 0.0324 mole) and the mixture was heated at 100°C for 2 hours. The reaction mixture was cooled in ice, diluted with ethyl alcohol (50 ml) and sodium borohydride (1.23 g, 0.0324 mole) was added and the mixture was stirred overnight at 25°C. The solvent was removed under reduced pressure and the residue was dissolved in ethyl acetate and the solution was washed with water and brine and then dried with sodium sulfate. The mixture was filtered and the solvent removed under reduced pressure to give N-(3-fluoro-4-methoxybenzyl)-aminoacetaldehyde dimethyl acetal as an oil (6.68 g, 85%).

To a solution of 1-(3-fluoro-4-methoxybenzyl)aminoacetaldehyde dimethyl acetal (6.68 g, 0.0275 mole) in ethyl alcohol (40 ml) was added potassium thiocyanate (2.94 g, 0.0302 mole) in water (65 ml) followed by 12 N hydrochloric acid (6 ml) and the reaction mixture was heated under reflux for 3 hours. The solvent was partially removed under reduced pressure and the mixture was cooled in ice and the product filtered. The product was recrystallized from ethyl alcohol to give 1-(3-fluoro-4-methoxybenzyl)-2-mercaptoimidazole as a solid melting at 156-157° (3.98 g, 61%).

Example 6

1-(3,5-Difluoro-4-methoxybenzyl)-2-mercaptoimidazole

A mixture of 3,5-difluoro-4-methoxybenzonitrile (3.50 g, 0.0207 mole) and Raney catalyst powder (3.5 g) in 90% formic acid (35 ml) was stirred and heated under reflux for 2.5 hours and the catalyst was filtered and washed with hot water and hexane. The hexane layer was separated and the aqueous solution was extracted an additional three times with hexane. The combined hexane extracts were washed with water and brine, dried and the solvent was removed to give 3,5-difluoro-4-methoxybenzaldehyde as an oil (3.38 g, 95%).

3,5-Difluoro-4-methoxybenzaldehyde (3.38 g, 0.0196 mole) was added to aminoacetaldehyde dimethyl acetal (2.14 ml, 0.0196 mole) and the mixture was heated at 100°C for 2 hours. The reaction mixture was cooled in ice, diluted with ethyl alcohol (35 ml), and sodium borohydride (0.743 g., 0.0196 mole) was added and the mixture was stirred overnight at 25°C. The solvent was removed under reduced pressure, the residue was dissolved in ethyl acetate, and the solution was washed with water and brine and then dried with sodium sulfate. The mixture was filtered, and the solvent removed under reduced pressure to give N-(3,5-difluoro-4-methoxybenzyl)aminoacetaldehyde dimethyl acetal as an oil (4.94 g, 96%).

To a solution of N-(3,5-difluoro-4-methoxybenzyl)aminoacetaldehyde dimethyl acetal (4.94 g, 0.0189 mole) in ethyl alcohol (30 ml) was added potassium thiocyanate (2.10 g, 0.0216 mole) in water (48 ml) followed by 12 N hydrochloric acid (4 ml), and the reaction mixture was heated under reflux for 3.5 hours. The solvent was partially removed under reduced pressure, the mixture was cooled in ice, and the product was filtered. The product was recrystallized from ethyl alcohol - hexane to give 1-(3,5-difluoro-4-methoxybenzyl)-2-mercaptoimidazole as a solid melting at 160-161° (2.22 g, 46%).

**Claims**

**Claims for the following Contracting States : AT, BE, DE, FR, GB, IT, LU, LI, CH, SE, NL**

**1.** A pharmaceutical composition comprising a pharmaceutically suitable carrier and a compound having the formula (I):

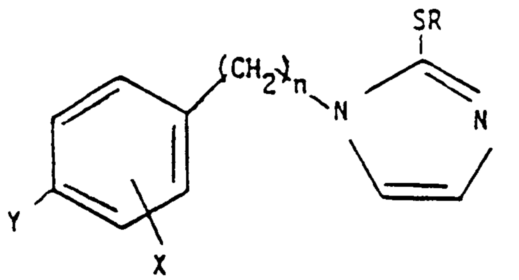

(I)

in which:

Y is $C_{1-4}$ alkoxy;

X is -H, -OH, halogen, $C_{1-4}$ alkyl, -CN, $-NO_2$, $-SO_2NH_2$, $-CO_2H$, $-CONH_2$, -CHO, $-CH_2OH$, $-CF_3$, $C_{1-4}$ alkoxy, $-SO_2C_{1-4}$ alkyl, $-SO_2C_{1-4}$ fluoroalkyl, or $-CO_2C_{1-4}$ alkyl or any accessible combination thereof up to four substituents;

R is -H or $C_{1-4}$ alkyl; and,

n is 1-4

or a hydrate or, when R is $C_{1-4}$ alkyl, a pharmaceutically acceptable acid addition salt thereof.

**2.** A composition of Claim 1 wherein the compound is 1-(3-fluoro-4-methoxybenzyl)-2-mercaptoimidazole or 1-(3,5-difluoro-4-methoxybenzyl)-2-mercaptoimidazole.

**3.** A compound of the formula (I): as described in claim 1 for the use as a therapeutic agent.

**4.** A compound of the formula (I): as described in claim 1 for the use as a dopamine $\beta$-hydroxylase inhibitor.

**5.** A compound that is 1-(3-fluoro-4-methoxybenzyl)-2-mercaptoimidazole or 1-(3,5-difluoro-4-methoxybenzyl)-2-mercaptoimidazole.

**6.** A process for preparing a pharmaceutical composition that comprises adding a pharmaceutically suitable carrier to a compound of the formula (I): as described in claim 1.

**7.** A process of Claim 6 wherein the compound is 1-(3-fluoro-4-methoxybenzyl)-2-mercaptoimidazole or 1-(3,5-difluoro-4-methoxybenzyl)-2-mercaptoimidazole.

**8.** A process for preparing 1-(3-fluoro-4-methoxybenzyl)-2-mercaptoimidazole that comprises reacting 1-(3-fluoro-4-methoxybenzyl)aminoacetaldehyde dimethyl acetal with acidic thiocyanate.

**9.** A process for preparing 1-(3,5-difluoro-4-methoxybenzyl)-2-mercaptoimidazole that comprises reacting 1-(3,5-difluoro-4-methoxybenzyl)aminoacetaldehyde dimethyl acetal with acidic thiocyanate.

**Claims for the following Contracting States : GR, ES**

**1.** A process for preparing a pharmaceutical composition that comprises adding a pharmaceutically suitable carrier to a compound of the formula (I):

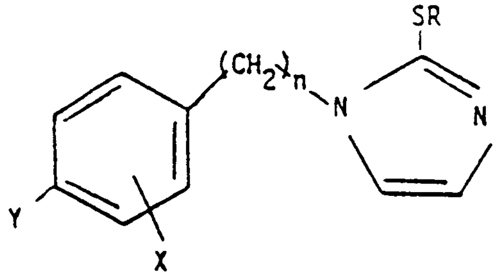

(I)

in which:

Y is $C_{1-4}$ alkoxy;

X is -H, -OH, halogen, $C_{1-4}$ alkyl, -CN, $-NO_2$, $-SO_2NH_2$, $-CO_2H$, $-CONH_2$, -CHO, $-CH_2OH$, $-CF_3$, $C_{1-4}$ alkoxy, $-SO_2C_{1-4}$ alkyl, $-SO_2C_{1-4}$ fluoroalkyl, or $-CO_2C_{1-4}$ alkyl or any accessible combination thereof up to four substituents;

R is -H or $C_{1-4}$ alkyl; and,

n is 1-4

or a hydrate or, when R is $C_{1-4}$ alkyl, a pharmaceutically acceptable acid addition salt thereof.

**2.** A process of Claim 1 wherein the compound is 1-(3-fluoro-4-methoxybenzyl)-2-mercaptoimidazole or 1-(3,5-difluoro-4-methoxybenzyl)-2-mercaptoimidazole.

**3.** A process for preparing 1-(3-fluoro-4-methoxybenzyl)-2-mercaptoimidazole that comprises reacting 1-(3-fluoro-4-methoxybenzyl)aminoacetaldehyde dimethyl acetal with acidic thiocyanate.

**4.** A process for preparing 1-(3,5-difluoro-4-methoxybenzyl)-2-mercaptoimidazole that comprises reacting 1-(3,5-difluoro-4-methoxybenzyl)aminoacetaldehyde dimethyl acetal with acidic thiocyanate.

**Revendications**

**Revendications pour les Etats contractants suivants : AT, BE, DE, FR, GB, IT, LU, LI, CH, NL, SE**

**1.** Composition pharmaceutique comprenant un véhicule acceptable du point de vue pharmaceutique et un composé de formule (I):

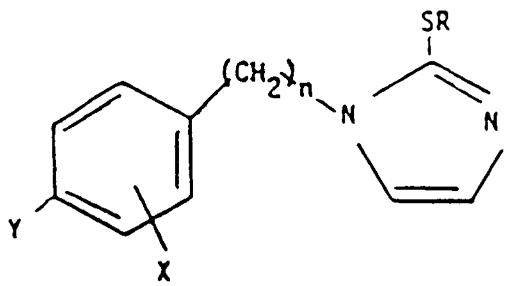

(I)

dans laquelle:

Y est un alkoxy en $C_{1-4}$;

X est -H, -OH, un halogène, un alkyle en $C_{1-4}$, -CN, $-NO_2$, $-SO_2NH_2$, $-CO_2H$, $-CONH_2$, -CHO, $-CH_2OH$, $-CF_3$, un alkoxy en $C_{1-4}$, un $-SO_2$-(alkyle en $C_{1-4}$), un $-SO_2$-fluoroalkyle en $C_{1-4}$), un $-CO_2$-(alkyle en $C_{1-4}$) ou l'une de leurs combinaisons accessibles jusqu'à quatre substituants;

R est -H ou un alkyle en $C_{1-4}$; et

n est un nombre entier de 1 à 4,

ou un hydrate ou, lorsque R est un alkyle en $C_{1-4}$, un de leurs sels d'addition d'acide acceptable du point de vue pharmaceutique.

**2.** Composition selon la Revendication 1, dans laquelle le composé est le 1-(3-fluoro-4-méthoxybenzyl)-2-mercaptoimidazole ou le 1-(3,5-difluoro-4-méthoxybenzyl)-2-mercaptoimidazole.

3. Composé de formule (I) selon la Revendication 1 destiné a être utilisé en tant qu'agent thérapeutique.

4. Composé de formule (I) selon la Revendication 1 destiné a être utilisé en tant qu'inhibiteur de la dopamine-$\beta$-hydroxylase.

5. Composé qui est le 1-(3-fluoro-4-méthoxybenzyl)-2-mercaptoimidazole ou le 1-(3,5-difluoro-4-méthoxybenzyl)-2-mercaptoimidazole.

6. Procédé de préparation d'une composition pharmaceutique qui comprend l'addition d'un véhicule acceptable du point de vue pharmaceutique à un composé de formule (I) selon la Revendication 1.

7. Procédé selon la Revendication 6 dans lequel le composé est le 1-(3-fluoro-4-méthoxybenzyl)-2-mercaptoimidazole ou le 1-(3,5-difluoro-4-méthoxybenzyl)-2-mercaptoimidazole.

8. Procédé de préparation du 1-(3-fluoro-4-méthoxybenzyl)-2-mercaptoimidazole qui comprend la réaction du 1-(3-fluoro-4-méthoxybenzyl)aminoacétaldéhyde diméthyl acétal avec un thiocyanate acide.

9. Procédé de préparation du 1-(3,5-difluoro-4-méthoxybenzyl)-2-mercaptoimidazole qui comprend la réaction du 1-(3,5-difluoro-4-méthoxybenzyl)aminoacétaldéhyde diméthyl acétal avec un thiocyanate acide.

**Revendications pour les Etats contractants suivants : GR, ES**

1. Procédé de préparation d'une composition pharmaceutique comprenant l'addition d'un véhicule acceptable du point de vue pharmaceutique à un composé de formule (I):

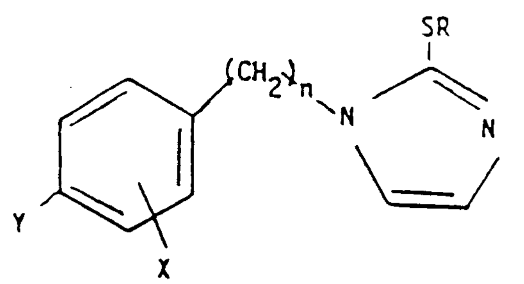

(I)

dans laquelle:

Y est un alkoxy en $C_{1-4}$;

X est -H, -OH, un halogène, un alkyle en $C_{1-4}$, -CN, $-NO_2$, $-SO_2NH_2$, $-CO_2H$, $-CONH_2$, -CHO, $-CH_2OH$, $-CF_3$, un alkoxy en $C_{1-4}$, un $-SO_2$-(alkyle en $C_{1-4}$), un $-SO_2$-(fluoroalkyle en $C_{1-4}$), un $-CO_2$-(alkyle en $C_{1-4}$) ou l'une de leurs combinaisons accessibles jusqu'à quatre substituants;

R est -H ou un alkyle en $C_{1-4}$; et

n est un nombre entier de 1 à 4,

ou un hydrate ou, lorsque R est un alkyle en $C_{1-4}$, un de leurs sels d'addition d'acide acceptable du point de vue pharmaceutique.

2. Procédé selon la Revendication 1, dans lequel le composé est le 1-(3-fluoro-4-méthoxybenzyl)-2-mercaptoimidazole ou le 1-(3,5-difluoro-4-méthoxybenzyl)-2-mercaptoimidazole.

3. Procédé de préparation du 1-(3-fluoro-4-méthoxybenzyl)-2-mercaptoimidazole qui comprend la réaction du 1-(3-fluoro-4-méthoxybenzyl)aminoacétaldéhyde diméthyl acétal avec un thiocyanate acide.

4. Procédé de préparation du 1-(3,5-difluoro-4-méthoxybenzyl)-2-mercaptoimidazole qui comprend la réaction du 1-(3,5-difluoro-4-méthoxybenzyl)aminoacétaldéhyde diméthyl acétal avec un thiocyanate acide.

## Patentansprüche

### Patentansprüche für folgende Vertragsstaaten : AT, BE, DE, FR, GB, IT, LU, LI, CH, NL, SE

1. Pharmazeutische Zusammensetzung, enthaltend einen pharmazeutisch geeigneten Trägerstoff und eine Verbindung der Formel (I)

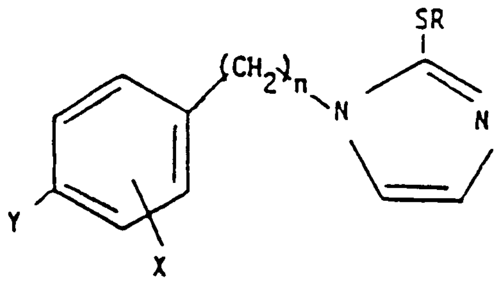

(I)

worin:
Y die Bedeutung $C_{1-4}$-Alkoxy hat;
X die Bedeutung -H, -OH, Halogen, $C_{1-4}$-Alkyl, -CN, $-NO_2$, $-SO_2NH_2$, $-CO_2H$, $-CONH_2$, -CHO, $-CH_2OH$, $-CF_3$, $C_{1-4}$-Alkoxy, $-SO_2C_{1-4}$-Alkyl, $-SO_2C_{1-4}$-Fluoralkyl, $-CO_2C_{1-4}$-Alkyl hat oder beliebige zugängliche Kombinationen davon mit bis zu vier Substituenten bedeutet;
R die Bedeutung -H oder $C_{1-4}$-Alkyl hat; und
n einen Wert von 1 bis 4 hat;
oder ein Hydrat oder, wenn R die Bedeutung $C_{1-4}$-Alkyl hat, ein pharmazeutisch verträgliches Säureadditionssalz davon.

2. Zusammensetzung nach Anspruch 1, wobei es sich bei der Verbindung um 1-(3-Fluor-4-methoxybenzyl)-2-mercaptoimidazol oder 1-(3,5-Difluor-4-methoxybenzyl)-2-mercaptoimidazol handelt.

3. Verbindung der Formel (I) nach Anspruch 1, zur Verwendung als therapeutisches Mittel.

4. Verbindung der Formel (I) nach Anspruch 1, zur Verwendung als Dopamin-$\beta$-hydroxylase-Inhibitor.

5. Verbindung, nämlich 1-(3-Fluor-4-methoxybenzyl)-2-mercaptoimidazol oder 1-(3,5-Difluor-4-methoxybenzyl)-2-mercaptoimidazol.

6. Verfahren zur Herstellung einer pharmazeutischen Zusammensetzung, umfassend die Zugabe eines pharmazeutisch geeigneten Trägerstoffs zu einer Verbindung der Formel (I) nach Anspruch 1.

7. Verfahren nach Anspruch 6, wobei es sich bei der Verbindung um 1-(3-Fluor-4-methoxybenzyl)-2-mercaptoimidazol oder 1-(3,5-Difluor-4-methoxybenzyl)-2-mercaptoimidazol handelt.

8. Verfahren zur Herstellung von 1-(3-Fluor-4-methoxybenzyl)-2-mercaptoimidazol, umfassend die Umsetzung von 1-(3-Fluor-4-methoxybenzyl)-aminoacetaldehyd-dimethylacetal mit saurem Thiocyanat.

9. Verfahren zur Herstellung von 1-(3,5-Difluor-4-methoxybenzyl)-2-mercaptoimidazol, umfassend die Umsetzung von 1-(3,5-Difluor-4-methoxybenzyl)-aminoacetaldehyd-dimethylacetal mit saurem Thiocyanat.

### Patentansprüche für folgende Vertragsstaaten : GR, ES

1. Verfahren zur Herstellung einer pharmazeutischen Zusammensetzung, umfassend die Zugabe eines pharmazeutisch geeigneten Trägerstoffs zu einer Verbindung der Formel (I)

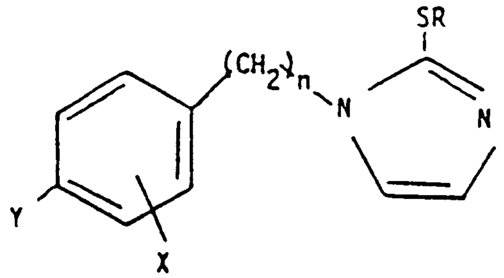

(I)

worin:

Y die Bedeutung $C_{1-4}$-Alkoxy hat;

X die Bedeutung -H, -OH, Halogen, $C_{1-4}$-Alkyl, -CN, $-NO_2$, $-SO_2NH_2$, $-CO_2H$, $-CONH_2$, -CHO, $-CH_2OH$, $-CF_3$, $C_{1-4}$-Alkoxy, $-SO_2C_{1-4}$-Alkyl, $-SO_2C_{1-4}$-Fluoralkyl, $-CO_2C_{1-4}$-Alkyl hat oder beliebige zugängliche Kombinationen davon mit bis zu vier Substituenten bedeutet;

R die Bedeutung -H oder $C_{1-4}$-Alkyl hat; und

n einen Wert von 1 bis 4 hat;

oder ein Hydrat oder, wenn R die Bedeutung $C_{1-4}$-Alkyl hat, ein pharmazeutisch verträgliches Säureadditionssalz davon.

**2.** Verfahren nach Anspruch 1, wobei es sich bei der Verbindung um 1-(3-Fluor-4-methoxybenzyl)-2-mercaptoimidazol oder 1-(3,5-Difluor-4-methoxybenzyl)-2-mercaptoimidazol handelt.

**3.** Verfahren zur Herstellung von 1-(3-Fluor-4-methoxybenzyl)-2-mercaptoimidazol, umfassend die Umsetzung von 1-(3-Fluor-4-methoxybenzyl)-aminoacetaldehyd-dimethylacetal mit saurem Thiocyanat.

**4.** Verfahren zur Herstellung von 1-(3,5-Difluor-4-methoxybenzyl)-2-mercaptoimidazol, umfassend die Umsetzung von 1-(3,5-Difluor-4-methoxybenzyl)-aminoacetaldehyd-dimethylacetal mit saurem Thiocyanat.